# EUROPEAN PATENT APPLICATION

(11) **EP 4 732 754 A1**
(43) Date of publication of application: **29.04.2026**
(21) Application number: 24208890.4
(22) Date of filing: 25.10.2024
(51) Int. Cl.: A61B 3/10

(54) **POLYGON SCANNING MIRROR MASK IN AN OPHTHALMIC IMAGING INSTRUMENT**

(71) Applicant: Optos plc, Dunfermline, Scotland KY11 8GR (GB)
(72) Inventor: THOMSON, Martin, Dunfermline, Scotland, KY11 8GR (GB); HOLLMANN, Stephen, Dunfermline, Scotland, KY11 8GR (GB); FIELD, Paul, Dunfermline, Scotland, KY11 8GR (GB)
(74) Representative: Hoffmann Eitle

(57) **Abstract**

An ophthalmic imaging instrument (102, 120) comprising a light source (200) which emits a beam of light (201), a polygon scanning mirror (202) comprising a plurality of reflecting facets (2021, 2022), a driver (203) which rotates during operation said polygon scanning mirror (202), such that each facet reflects the beam of light (201), an optical system (220) which guides the beam reflected from the polygon scanning mirror (202) towards a fundus (F) of an eye (E) through a cornea (C) and guides light (LR) returning from the fundus (F) towards the polygon scanning mirror (202), a mask (206) which blocks a portion (P201) of the beam (201) of light which has been reflected back from at least one of the cornea (C) and an optical element (204, 206, 208) of the optical system from reaching a detector (205), the detector (205) arranged to detect the returning light (LR) to generate a detection signal (Sd), and an access (207) to computing resources (106) which is arranged to generate an image (210) of the fundus (F) of the eye (E) using the detection signal (Sd).

## Description

### Technical field

The present invention relates to a polygon scanning mirror in an ophthalmic imaging instrument. More particularly but not exclusively, the present invention relates to a mask arranged to block a portion of the beam reflected from the polygon scanning mirror which has been reflected back from at least one of a cornea of the eye and an optical element of an optical system of the ophthalmic imaging instrument from reaching a detector of the ophthalmic imaging instrument.

### Background

Ophthalmic imaging instruments are widely used to image a patient's eye in order to assess the health of the eye. Such instruments typically comprise a light source and a controller that is arranged to control the light source to generate a beam of light of a target optical power. The beam of light is deflected by one or more scan relay elements so as to cover an imaging area by means of a typically parallel arrangement of a plurality of scanning lines. The scan relay element(s) usually comprise a polygon scanning mirror and one or more scanning galvanometer scanning mirrors, herein referred to as "galvos". The former polygon scanning mirror comprises a plurality of reflecting facets that are arranged along an outer circumference of a rotating body. A driver is operable to rotate the body of the polygon scanning mirror, such that each facet successively reflects an incident beam of light at a varying angle. Thus, during the passing of one individual facet, the beam of light is deflected through the varying angle. After completion of such a deflection cycle, the next facet substantially repeats this process and so do all other facets.

This repeated deflection of the beam of light by the polygon scanning mirror forms the basis for at least one scanning direction by means of providing a plurality of parallel scanning lines. A further scanning element, such as an already mentioned galvo, can then further deflect the beam in another direction so as to displace the scanning lines from each other on the target and to ultimately cover a two-dimensional scanning area. At least in some imaging modalities, the light from the scanning beam is scattered back from the target tissue which includes in the case of ophthalmic imaging primarily parts of the human eye such as, for example, the fundus or retina. In general, the light thus travels back along the incident light path so as to be ultimately detected by a detector in the form of a light sensor, converted into an intensity signal, and processed to form the image. Especially the latter signal processing is implemented by means of computing resources that compile individual images from corresponding sets of line scans.

Where the target tissue is the fundus of the eye, the light from the scanning beam passes through the cornea of the eye. The cornea (e.g. the outer surface of the cornea) may specularly reflect some of the scanning beam back towards the detector, thereby forming an image artifact in the image. Other image artifacts may be similarly formed from some of the scanning beam specularly reflecting back from optical elements within the ophthalmic imaging instrument.

Therefore, there is a need to suppress or eliminate the image artifacts formed by these back-reflections of the scanning beam, whilst keeping system complexity at an acceptable level.

### Summary

According to one embodiment of the present invention, there is provided an ophthalmic imaging instrument. The ophthalmic imaging instrument comprises a light source, a polygon scanning mirror, a driver, an optical system, a mask, a detector and an access to computing resources. The light source is arranged to emit a beam of light. The polygon scanning mirror comprises a plurality of reflecting facets. The driver is arranged to rotate during operation said polygon scanning mirror, such that each facet reflects the beam of light at a varying angle. The optical system is arranged to guide the beam reflected from the polygon scanning mirror towards a fundus of an eye of a subject through a cornea of the eye and to guide light returning from the fundus towards the polygon scanning mirror. The mask is arranged to block a portion of the beam reflected from the polygon scanning mirror which has been reflected back from at least one of the cornea of the eye and an optical element of the optical system from reaching the detector. The detector is arranged to detect the returning light reflected from the polygon scanning mirror to generate a detection signal. The access is to computing resources which are arranged to generate an image of the fundus of the eye using the detection signal.

According to a further embodiment of the present invention there is provided a method for operating the ophthalmic imaging instrument described above. The method comprises a step of rotating, by means of the driver, said polygon scanning mirror such that each facet reflects the beam of light, a step of guiding, by means of the optical system, the beam reflected from the polygon scanning mirror towards the fundus of the eye of the subject, a step of guiding, by means of the optical system, light returning from the eye towards the polygon scanning mirror, a step of blocking, by means of the mask, the portion of the beam reflected from the polygon scanning mirror from reaching the detector, a step of generating, by means of the detector, the detection signal by detecting the returning light, and a step of generating, by means of the access to the computing resources, the image of the fundus of the eye using the detection signal.

### Brief Description of the Drawings

Embodiments of the present invention, which are presented for better understanding of the inventive concepts, but which are not to be seen as limiting the invention, will now be described with reference to the figures in which:
- Figure 1: shows a schematic view of an ophthalmic imaging instrument according to a general device embodiment of the present invention;
- Figure 2: shows a schematic view of an ophthalmic imaging instrument according to a device embodiment of the present invention;
- Figure 3: shows an enlarged schematic view of the rotating polygon mirror and the mask according to the device embodiment of the present invention to illustrate certain further aspects therein;
- Figure 4: shows a schematic view of an ophthalmic imaging instrument especially in the context of examples of the optical system according to an embodiment of the present invention;
- Figure 5: shows a flowchart of a general method embodiment of the present invention.

It should be understood that some of the drawings may not necessarily be shown to scale, unless otherwise indicated. In certain instances, details that are not necessary for an understanding of the disclosure or that render other details difficult to perceive may have been omitted. It should be understood, of course, that the disclosure is not necessarily limited to the particular examples or embodiments illustrated or depicted herein.

### Detailed Description

The present inventors have recognised that specular reflections of the scanning beam from the cornea and from optical elements of an optical system within the ophthalmic imaging instrument may follow an optical path which differs to that of both the scanning beam and the returning light from the eye. Accordingly, the inventors have devised a mask which blocks such portions of the scanning beam which have been reflected back from at least one of the cornea of the eye and an optical element of the optical system of ophthalmic imaging instrument from reaching a detector of the ophthalmic imaging instrument. The mask may thereby at least partially suppress image artifacts in images of the fundus which would otherwise result from the detection of these back-reflections. The quality of images generated by the ophthalmic imaging instrument may thus be greatly improved.

Example embodiments herein will now be described with reference to the accompanying drawings.

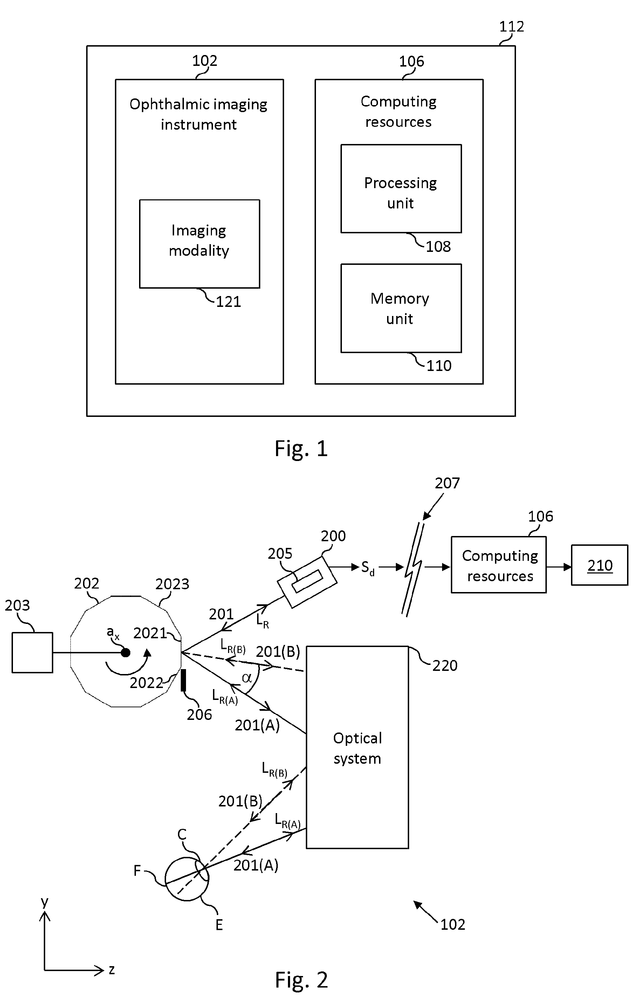

Figure 1 shows a schematic view of an ophthalmic imaging instrument 102 according to a general device embodiment of the present invention. Specifically, there is shown an ophthalmic imaging system 112 which includes the ophthalmic imaging instrument 102 providing at least one imaging modality 121 such as, for example, autofluorescence (AF), colour red-green, colour red-green-blue and fluorescein angiography. The ophthalmic imaging instrument 102 may be, or comprise, a scanning laser ophthalmoscope (SLO) so as to provide one or more of the at least one imaging modalities 121 and may comprise an optical coherence tomography (OCT) device so as to provide an OCT imaging modality. In some embodiments, the OCT device may share one or more optical components with the SLO such as, for example, the scanning system therein. The ophthalmic imaging system 112 may comprise or have access to (e.g. via a wired or wireless connection) computing resources 106 which, in turn, comprise a processing unit 108 and a memory unit 110. The components of the ophthalmic imaging system 112 including the ophthalmic imaging instrument 102 and the computing resources 106 may be housed inside a common housing so that the system 112 thereby forms the ophthalmic imaging instrument 102, such as an ophthalmoscope. In this way, the ophthalmic imaging instrument 102 has access to, or itself comprises, the computing resources 106. In some embodiments, the computing resources 106 may be located external to the instrument 102 within respective different housings. In some embodiments, any of the components may be located in a different housing from the rest of the components.

The computing resources 106 are operable to generate an image of a fundus of the eye using a detection signal from a detector in the ophthalmic imaging instrument 102, as later described, and may be operable to control and provide the imaging modality 121.

The computing resources have at least one processing unit 108 such as a central processing unit (CPU) and/or graphics processing unit (GPU), and a memory unit 110 that stores instructions that, when executed by the at least one processing unit 108, causes the processing unit 108 to perform one or more methods and functions described herein. In embodiments of local computing resources or local physical components of a computing device, the resources may include a processor, such as CPU and/or GPU, a system memory, and a system bus that couples the system memory to the CPU/GPU. The system memory may include a random access memory ("RAM") and a read-only memory ("ROM"). A basic input/output ("I/O") system containing the basic routines that help to transfer information between elements within the computing device, such as during startup, is stored in the ROM. The computing resources may further include a mass storage device, which is able to store software instructions and data. The mass storage device may be connected to the CPU/GPU through a mass storage controller connected to the system bus. The mass storage device and its associated computer-readable data storage media may provide non-volatile, non-transitory storage for the computing resources 106. Although the description of computer-readable data storage media contained herein refers to a mass storage device, such as a hard disk or CD-ROM drive, it should be appreciated by those skilled in the art that computer-readable data storage media can be any available non-transitory, physical device or article of manufacture from which the device can read data and/or instructions. The mass storage device is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the computing device.

The computing resources 106 may operate in a networked environment using logical connections to remote network devices through a network, such as a local network, the Internet, or another type of network. The computing resources 106 may connect to the network through a network interface unit connected to the system bus. The network interface unit may also connect to other types of networks and remote computing systems. The computing resources 106 may include an input/output controller for receiving and processing input from a number of other devices, including a touch user interface display screen, or another type of input device. Similarly, the input/output controller may provide output to a touch user interface display screen, a printer, or other type of output device. As mentioned above, the mass storage device and the RAM can store software instructions and data. The software instructions may include an operating system suitable for controlling the operation of the computing resources 106. The mass storage device and/or the RAM may also store software instructions, that when executed by the CPU/GPU, cause the computing resources 106 to provide the functionality discussed in this document, including the methods described herein and shown in the figures.

In some embodiments which implement imaging modalities 121 with the mentioned SLO, the SLO may be a confocal laser scanning microscope for diagnostic imaging of the retina of the eye. The imaging may be two-dimensional (2D) imaging and may use a laser light beam to scan across the retina in a raster pattern to illuminate successive elements of the retina, point-by-point. The light reflected from each retinal point may be captured by a photomultiplier. The output of the photomultiplier may be recorded and displayed in a digital format. In this manner, the SLO may be able to produce high-contrast, detailed images of the retina. In some embodiments, images are captured sequentially by one imaging modality using the SLO and by at least a further imaging modality using at least one of the SLO and the OCT device.

Figure 2 shows a schematic view of the ophthalmic imaging instrument 102 according to a device embodiment of the present invention. As shown, the ophthalmic imaging instrument 102 comprises a light source 200 arranged to emit a beam of light 201 as a scanning beam. A polygon scanning mirror 202 acts a first scanning element (or, in other terms, a first scan relay) which comprises a plurality of reflecting facets 2021, 2022, ... and is arranged to rotate in a plane of rotation (i.e. parallel to the y-z plane) about an axis of rotation a_{X}. The facets may each have the same shape and may each extend a distance in a common direction (i.e. the x-direction) orthogonal to the plane of rotation of the polygon scanning mirror 202. In some embodiments, the beam 201 may have a cross-sectional area which is fraction of the area of the facets (e.g. less than 0.1 or 1% of the area of each of the facets). The polygon scanning mirror 202 is arranged to reflect the beam 201 towards an optical system 220 and to reflect returning light L_{R} from the eye E towards the detector 205, as later described. As shown, the beam 201 may, upon incidence with the polygon scanning mirror 202, be travelling with a component in the direction opposite to the positive y-direction. In the present embodiment, the polygon scanning mirror is a regular convex polygon with 12 rectangular facets, each having a length defined by the lengths of two edges of the polygon scanning mirror 202 parallel to the plane of rotation of the polygon scanning mirror 202 and a width in the common direction defined by the lengths of two edges of the polygon scanning mirror 202 orthogonal to this plane. However, other numbers of facets (e.g. 16 facets or at least four or five facets), shapes of facets or geometries of polygon may instead be used.

A driver 203 is arranged to rotate during operation said polygon scanning mirror 202 (in the plane of rotation of the polygon scanning mirror 202) such that each facet 2021, 2022, ... reflects the beam of light 201 at a varying angle α (when the facet is rotated over a predetermined range of angular positions). The driver 203 may, for example, be an electric motor connected to the polygon scanning mirror 202 and controlled by a controller of the ophthalmic imaging instrument 102 (which may be the computing resources 106). As illustrated, the path of the scanning beam 201 is shown in a one-dimensional (1D) scan produced during example anti-clockwise rotation (shown by the curved arrow) of the polygon scanning mirror 202. Path "A" is an example of the scanning beam 201 reflected from a respective facet 2021 of the polygon scanning mirror 202 at a starting point during rotation, i.e. when the facet 2021 is moved into the path of the beam 201 from the light source 200. Path "B" is an example of the scanning beam 201 reflected from the respective facet 2021 of the polygon scanning mirror 202 at a later point in time further down the rotation when the facet 2021 is moved out of the path of the beam 201 from the light source 200.

In this way, the polygon scanning mirror 202 reflects the beam 201 at (or through) a varying angle α from the active facet (i.e. the facet presently reflecting the beam 201 from the light source 200 to the optical system 220) as the beam 201 moves from one edge of the facet defining the facets length to the other. As the facet 2021 moves out of reach of the beam 201, the adjacent facet 2022 (i.e. the neighbouring/bordering facet to the facet 2021 in the opposite rotational direction to that of the polygon scanning mirror 202) repeats substantially the described reflection process such that each facet successively reflects the beam 201 at the varying angle α in turn. This varying angle α varies as governed by the length and rotation of the respective facet and the respective orientation of the facet relative to the incident beam 201 determines the reflection angle of the beam 201.

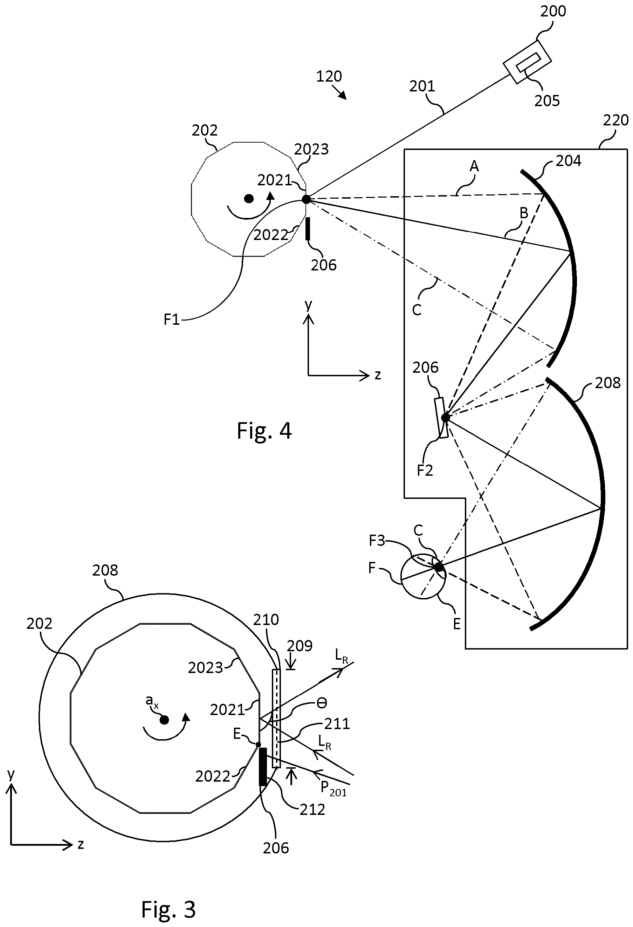

The ophthalmic imaging instrument 102 further comprises the optical system 220 arranged to guide the beam of light 201 that is reflected from the polygon scanning mirror 202 (i.e. the beam 201 reflected at the varying angle α) towards a fundus F of an eye E of a subject through a cornea C of the eye E. Examples of components and optical elements within the optical system 220 are described later with reference to Figure 4.

The optical system 220 is further arranged to guide light L_{R} returning from the fundus F towards the polygon scanning mirror 202. The returning light L_{R} may be light scattered from the eye E in, for example, the colour red-green or the colour red-green-blue imaging modality or may be, or comprise, light emitted from the eye E in, for example, the AF imaging modality. This return light L_{R} may follow the same optical path as the beam 201 and so may be guided by the optical system 220 towards the polygon scanning mirror 202 along optical paths corresponding to the reflection of the beam 201 at the varying angle α, as later described. The returning light L_{R} is thus guided by the optical system 220 to the active facet and this active facet reflects the returning light L_{R} towards a detector 205 along the optical path of the beam 201 so as to form an image 210 of the eye E, as later described. As the current active facet moves out of reach of the beam 201, the adjacent facet becomes the active facet and repeats substantially this described reflection process of the returning light L_{R} such that each facet reflects the returning light L_{R} towards the detector 205 along the optical path of the beam 201, in turn. Thus, successive scan lines in the image 210 may be formed by using, for example, an orthogonal scanning element. As later described, in some embodiments these successive scan lines in the image 210 may correspond to a part β of the varying angle α which may be different to the varying angle α and, more specifically, be a subset of it (e.g. greater than 50, 70 or 90% and less than 95 or 100% of the varying angle α, either centred within the varying angle α or from the start of the varying angle α where the active facet first reflects the beam 201).

In some embodiments, the returning light L_{R} from the eye E may be a light beam of a diameter which is greater than that of the beam 201 and which may be (substantially) equal to that of the pupil of the eye E. The light beam may be of a size which (substantially) fills the entire area of each of the facets . However, it would be appreciated by those skilled in the art that this depends on the components and optical elements in the optical system 220, the characteristics of the beam 201 and the imaging modality 121, for example.

As previously described, some of the beam 201 that is reflected from the polygon scanning mirror 202 and guided towards the eye E by the optical system 220 may be reflected back from the cornea C so as to form image artifacts in the image 210. Similarly, some of the beam 201 that is reflected from the polygon scanning mirror 202 may be reflected back from at least one of the optical elements in the optical system 220 so as to also form image artifacts in the image 210. These back-reflected parts of the beam 201 may follow a different optical path to the returning light L_{R} to be incident on the detector 205 and so, as recognised by the inventors, may be blocked from reaching the detector 205 without, or while minimally, blocking/obstructing the returning light L_{R} used to generate the image 210.

Accordingly, the ophthalmic imaging instrument 102 further comprises a mask (or light blocker) 206 arranged to block a portion of the beam of light 201 that is reflected from the polygon scanning mirror 202 which has been reflected back (i.e. back-reflected) from at least one of the cornea C of the eye E and/or an optical element of the optical system 220 (along an optical path which would be detected by the detector 205) from reaching the detector 205 (e.g. via the polygon scanning mirror 202, as set out below). The mask 206 may achieve this by at least one of: substantially absorbing the portion of the beam 201 (e.g. absorbing at least 90 or 95% of the portion of the beam 201); and/or reflecting the portion of the beam 201 along an optical path which is not incident on the detector 205 (so that it is not detected by the detector 205). Thereby, the mask 206 may prevent the portion of the beam 201 from reaching the detector. This portion of the beam of light 201 is a part of the outgoing scanning beam 201 which is reflected back towards the detector 205 without having propagated to the fundus F (and so is therefore not a part of the returning light L_{R}). As shown, the mask 206 is arranged between the rotating polygon mirror 202 and the optical system 220, although other arrangements are possible as later described. Where the mask 206 is arranged to substantially absorb the portion of the beam 201, as in the present embodiment, the mask 206 may be a non-reflective element such as, for example, a metal plate with an anti-reflection coating (e.g. an absorptive anti-reflection coating or a multilayer anti-reflection coating).

Figure 3 shows an enlarged schematic view of the rotating polygon mirror 202 and the mask 206 to illustrate further aspects of this arrangement. These components are shown alongside additional optional elements which may form a part of the ophthalmic imaging instrument 102, as set out below. Only the returning light L_{R} is shown so as to more clearly illustrate some aspects of the figure. An example is shown of an optical path of a portion P₂₀₁ of the beam 201 which has been reflected back from at least one of the cornea C of the eye E and/or an optical element of the optical system 220 which may be detected by the detector 205 and so is to be blocked by the mask 206, the optical path being different to that of the returning light L_{R}. However, it would be appreciated that other such portions to be blocked by the mask 206 (in the shown arrangement or in later-described arrangements of the mask 206) may exist along different optical paths to that of the shown portion P₂₀₁ of the beam 201 and that these portions, and the portion P₂₀₁, of the beam 201 may have a spatial distribution (not shown) about their optical path as determined, for example, by the element from which they are back-reflected. The angle between the optical path of the blocked portion P₂₀₁ of the beam 201 and the returning light L_{R} may vary during rotation of the polygon scanning mirror 202, as would be appreciated by those in the art.

The portion P₂₀₁ of the beam 201 is blocked by the mask 206 from being incident on the facet 2022 of the polygon scanning mirror 202, which is the next to reflect the beam 201 at the varying angle α during rotation of the polygon scanning mirror 202, for all optical paths of the portion P₂₀₁ of the beam 201 which would be incident on the facet 2022 during rotation of the polygon scanning mirror 202 whereby the detected returning light L_{R} forms the image 210. That is, where the portion P₂₀₁ of the beam 201 travels along any optical path which would be incident at any point on the facet 2022, it is blocked by the mask 206. Thereby, where each scan line of the image 210 is formed from the detected returning light L_{R} corresponding to a part β of the varying angle α extending to the present angular rotation of the polygon scanning mirror 202 as shown in Figure 3 (i.e. where the facet 2022 is parallel to the plane 211, as later described), the portion P₂₀₁ of the beam 201 is blocked by the mask 206 from being incident anywhere on the whole of facet 2022 so as to prevent any such portion P₂₀₁ of the beam 201 from reaching the detector 205, being detected and so forming an image artifact in the image 210. In such a case, the returning light L_{R} is thus blocked by the mask 206 from incidence on the facet 2022 during rotation of the polygon scanning mirror 202 whereby the detected returning light L_{R} forms the image 210. This allows the mask 206 to block any optical path of the portion P₂₀₁ of the beam 201 which is different to, and beneath, that of the returning light L_{R}, which may therefore greatly reduce the prevalence of image artifacts in the image 210 due to back-reflections of the beam 201. It would be appreciated however that if the rotating polygon mirror 202 rotates clockwise, the facet 2022 of the polygon scanning mirror 202 would be the facet which last reflected the beam 201 at the varying angle α during rotation of the polygon scanning mirror 202.

In some embodiments, the portion P₂₀₁ of the beam 201 is more generally blocked by the mask 206 from being incident on a portion of the facet 2022 of the polygon scanning mirror 202 during rotation of the polygon scanning mirror 202 whereby the detected returning light L_{R} forms the image 210. That is, where the portion P₂₀₁ of the beam 201 travels along an optical path which would be incident on the portion of the facet 2022, it is blocked by mask 206. For example, where each scan line of the image 210 is formed from the detected returning light L_{R} for the whole of the varying angle α shown in Figure 3, the portion P₂₀₁ of the beam 201 is blocked by the mask 206 from being incident on a varying portion of the facet 2022 as the polygon scanning mirror 202 rotates so as to prevent this portion P₂₀₁ of the beam 201 from reaching the detector 205, being detected and so forming an image artifact in the image 210. Therefore, image artifacts in the image 210 due to back-reflections may be at least partially reduced whilst utilising the whole of the varying angle α for the image 210, allowing an extended field of view of the ophthalmic imaging instrument 102 to be obtained (via utilising the whole of each facet's length when reflecting the beam 201).

As shown in Figure 3, a (e.g. flat) surface 212 of the mask 206 may block the portion P₂₀₁ of the beam 201, whereby each facet of the polygon scanning mirror 202 is parallel to the surface 212 of the mask 206 at a given angular rotation of the polygon scanning mirror 202. The present inventors have recognised that this vertical orientation of the surface of the mask relative to the facets is particularly advantageous at blocking the portion P₂₀₁ of the beam 201 by reflecting it along an optical path which is not incident on the detector 205. However, it would be appreciated that other orientations of the surface 212 of the mask 206 may instead be used.

As further shown in Figure 3, the facet 2022 shares an edge E (i.e. an edge along the x-direction) with the active facet 2021 presently reflecting the beam of light at the varying angle α, an obtuse angle θ being formed between the beam 201 and a line from the edge E to the point of incidence of the beam 201 on the active facet 2021. In other words, the facet 2022 shares the edge E of the active facet 2021 which is in the direction along the active facet 2021 of a projection of the beam 201 onto an orthogonal edge of the active facet 2021 to the edge E. In this way, the facet 2022 which the mask 206 prevents the portion P₂₀₁ of the beam 201 from being incident on is that beneath the active facet 2022 in Figure 3 (i.e. that sharing the edge E of the active facet 2021 further in the direction opposite the y-direction) during rotation of the polygon scanning mirror 202 whereby the detected returning light L_{R} forms the image 210.

This arrangement of blocking the facet 2022 beneath the active facet 2021 in Figure 3 has been recognised by the inventors to be particularly advantageous at suppressing image artifacts in the image 210 that would be formed by back-reflected portions of the beam 201 which would otherwise reach and so be detected by the detector 205. This is as the angle of this neighbouring facet 2022 relative to the returning light L_{R} means that stray back-reflections from the cornea C or optical element(s), if incident on the facet 2022, may be much more likely to return to the detector 205 than if, for example, incident on the upper facet 2023. This is especially the case where a housing encloses the polygon scanning mirror 202, as set out below, as back-reflections incident on facet 2023 will typically propagate so as to be contained within the housing. Where the rotating polygon mirror 202 has many more facets (e.g. 16 or more facets), the present inventors have further recognised that the aforementioned benefits of blocking the facet 2022 beneath the active facet 201 are further improved due to the angle of this facet 2022 relative to the returning light L_{R} approaching that of the active facet 2021 relative to the returning light L_{R} as the number of facets of the rotating polygon mirror 202 increases. However, in some embodiments, the mask 206 may alternatively be arranged to block the facet 2023 above the active facet 2022 from a portion of the beam 201 which has been reflected back from at least one of the cornea C of the eye E and an optical element of the optical system 220 (along an optical path which would be detected by the detector 205) from reaching the detector 205 (e.g. along an optical path which would be incident on the facet 2023 and may be different to that of the retuning light L_{R}).

The mask 206 may, as in the present device embodiment, extend a distance in the direction orthogonal to the plane of rotation of the polygon scanning mirror 202 (not shown) which is equal to or greater than the distance each facet extends in the direction orthogonal to the plane of rotation of the polygon scanning mirror 202. In this way, portion P₂₀₁ of the beam 201 is blocked by the mask 206 from incidence on the facets along the distance extended by the mask 206 in this direction. For example, the mask 206 may be a rectangular element (which comprises the surface 212) of a width that is equal to or greater than the width of the rectangular facets 2021, 2022, ... that are shown in Figure 3. This optional feature results the portion P₂₀₁ of the beam 201 being blocked by the mask 206 across the full width of each facet, which results in a further suppression of image artifacts in the image 210 that would be formed by back-reflections of the beam 201 reaching, and being detected by, the detector 205.

Whilst the mask 206 is described above to block either facet 2022, 2023 adjacent to the active facet 2021, the present invention is not so limited and the mask 206 may, more generally, block a portion of the beam of light 201 which has been reflected back from at least one of the cornea C of the eye E and/or an optical element of the optical system 220 towards the detector 205 from being incident on at least one facet of the polygon scanning mirror 202 that is not presently reflecting the beam of light 201 (i.e. at least one facet which is not the active facet) during rotation of the polygon scanning mirror 202 whereby the detected returning light L_{R} forms the image 210. That is, where the portion of the beam of light 201 travels along an optical path which would be incident on the at least one facet of the polygon scanning mirror 202, it is blocked by the mask 206. By way of example, where the housing 208 is removed or the opening 209 of the housing 208 is sufficiently large, the mask 206 may extend in the negative y-direction to further block the portion of the beam of light 201 from being incident on the next two facets to the facet 2022 in the clockwise direction should the back-reflected portion of the beam of light 201 follow an optical path onto either of these facets and towards the detector 205. Returning to Figure 3, the ophthalmic imaging instrument 102 further comprises an optional housing 208 with an opening 209, the housing 208 enclosing the rotating polygon mirror 202. As shown, the opening 209 may extend across a plane 211 (i.e. parallel to the x-y plane). The housing 208 may protect the ophthalmic imaging instrument 102 from failure of the fast-spinning polygon scanning mirror 202 by containing pieces of the polygon scanning mirror 202 which may be propelled outwards at high velocities in such instances. Where the optional housing 208 is provided, the mask 206 is fixed within the housing 208. However, where the housing 208 is omitted, the mask 206 may be otherwise attached to the ophthalmic imaging instrument 102 such as, for example, by being fixed to the aforementioned housing of the ophthalmic imaging instrument 102. The opening 209 may, as in the present device embodiment, be larger than the size of each facet of the plurality of facets so as to not, or negligibly, constrain (or limit) the returning light L_{R} reaching the detector 205 that is reflected from each (active) facet. However, in some embodiments, the mask 206 may be provided by way of the opening 209 and so may be implemented directly by the housing 208 so as to reduce the ophthalmic imaging instrument's component count.

As shown in Figure 3, the line orthogonal to the plane 211 and passing the centre of the plane 211 crosses the axis of rotation a_{X} of the polygon scanning mirror 202 and is parallel to the plane of rotation of the polygon scanning mirror 202. Further, each facet when reflecting the beam 201 is parallel to the plane 211 at a given rotation of the polygon scanning mirror 202. The portion P₂₀₁ of the beam 201 may, as in the present embodiment, be blocked by the mask 206 from being incident on the facet 2022 by the mask 206 blocking the opening 209 up to the edge E when the active facet 2022 is parallel to the plane 211. That is, the mask 206 extends parallel the plane 211 of the opening 209 up to the edge E so as to block the portion P₂₀₁ from incidence on the facet 2022 when the active facet 2021 is parallel to the plane 211. The surface 212 of the mask 206 may be parallel to the plane 211 of the opening 209, as shown. In this way, the mask 206 blocks all of the optical paths of the portion P₂₀₁ of beam 201 which would be incident on the facet 2022 during rotation of the polygon mirror 202 whereby the detected returning light L_{R} forms the image 210, as previously described.

The ophthalmic imaging instrument 102 (or, more specifically, the opening 209) further comprises an optional phase mask 210 which may be arranged to cover the opening 209. The phase mask 210 may correct for aberrations caused by at least one component and optical element of the optical system 220 (e.g. scanner surfaces and ellipsoidal mirrors, as later described).

Returning to Figure 2, the detector 205 is arranged to detect the returning light L_{R} reflected from the polygon scanning mirror 202 to generate a detection signal S_{d}. The detection signal S_{d} used may be that generated from the returning light L_{R} during rotation of the polygon scanning mirror 202 corresponding to the part β of the varying angle α for at least one facet although, as previously described, this is not necessarily the case. The detection signal S_{d} is transmitted to, and received by, the computing resources 106 via an access 207 to the computing resources 106. The detector 205 may comprise a one- or two-dimensional array of photo-sensing elements. However, the form of the detector 205 is not limited and the detector 205 may, for example, comprise a balanced photodetector arrangement comprising two reverse-biased photodiodes whose output photocurrents are subtracted from one another, the subtracted current signal being converted to a voltage detection signal by a transimpedance amplifier.

The access 207 is to the computing resources 106, wherein the computing resources 106 are arranged to generate an image 210 of the fundus F of the eye E using the detection signal S_{d}. The image 210 may, as in the present device embodiment, be generated using the detection signal S_{d} generated from the returning light L_{R} reflected by at least one facet (e.g. all facets) during rotation of the polygon scanning mirror 202. Thereby, the image 210 of the eye E is formed by successive scan lines each corresponding to the detection signal S_{d} generated from the returning light L_{R} detected during rotation of the polygon scanning mirror 202 corresponding to at least the part β of the varying angle α for each of the facets.

By blocking the portion P₂₀₁ of the beam 201 from reaching the detector 205, the mask 206 is therefore arranged to block the portion P₂₀₁ of the beam 201 so as to at least partially suppress (or remove) an image artifact in the image 210 of the fundus F which would result from a detection by the detector 205 of the portion P₂₀₁ of the beam 201 (e.g. via the polygon scanning mirror 202, as set out above). The quality of images generated by the ophthalmic imaging instrument 102 may thus be greatly improved, as previously described.

Whilst the mask 206 is described above to be arranged between the rotating polygon mirror 202 and the optical system 220 and to block the portion of the beam 201 from reaching the detector 205 via the polygon scanning mirror 202, the present invention is not so limited and the mask 206 may be placed at alternative locations within the ophthalmic imaging instrument 102. The placement of the mask 206 within the ophthalmic imaging instrument 102 may be chosen to at least partially suppress the aforementioned image artifact by: acquiring an image of the fundus F; identifying an image artifact in the image which is suspected to have resulted from back-reflections from at least one of the cornea C of the eye E and/or an optical element of the optical system 220; placing the mask 206 within the ophthalmic imaging instrument 102 at a first location (which may not, or negligibly, block the returning light L_{R}); acquiring a further image of the fundus F; and identifying whether the image artifact has been at least partially suppressed. If not, the mask 206 may be moved to a second location and so on until the image artifact has been at least partially suppressed. The mask 206 may thus be placed at the resulting location which at least partially supresses the image artifact, although it may be further moved to improve the degree by which the image artifact is supressed in the image. It would be appreciated by those skilled in the art that this iterative process can be quickened by identifying potential optical paths different to that of the returning light L_{R} which portions of the beam 201 might follow when back-reflected by the at least one of the cornea C of the eye E and the optical element of the optical system 220 so as to reach the detector 205 (e.g. by using ray-tracing software) and starting the iterative process by placing the mask 206 at locations which block these optical paths (as these locations are most likely to suppress the image artifact).

Figure 4 shows a schematic view of an ophthalmic imaging instrument 120 especially in the context of examples of the optical system 220 according to an embodiment of the present invention and there are again shown elements as explained amongst others in conjunction with Figure 2 (same reference signs thus denote same elements and functionalities). The optional component shown in Figure 3 may be further included, as explained above. The ophthalmic imaging instrument 120 is described in the context of an imaging modality in which a beam of light 201 is generated by the light source 200 and guided toward a patient's eye E, reflected at tissue of the same and guided back to a detector 205. As part of an SLO module, the instrument 120 includes the light source 200 emitting a beam of light, that is, a scanning beam 201, a polygon scanning mirror 202 (in other terms, a first scanning element 202) and an example of the optical system 220 comprising a plurality of further scan relay elements. The scan relay elements include a second scanning element 206 and optical elements that are positioned and configured to direct the scanning beam 201. In some embodiments, the optical elements may include the optical element 204 which may be a scan compensation element such as a curved mirror or slit mirror, and a second optical element 208 which may be a scan transfer element such as a main mirror.

The optical elements 204 and 208 are positioned and configured to direct the scanning beam 201. The second scanning element 206 may be or include an oscillating plane scanning mirror or a planar scanning mirror coupled to a galvanometer motor. The optical element 204 may be a curved mirror such as an ellipsoidal mirror. The second optical element 208 may be an aspherical mirror. It is to be appreciated that the first and second optical elements may have an alternative form. The scanning elements 202 and 206 may be referred to as a scanning device or a plurality of individual scanning devices. It is to be understood that the shown functionalities are just an example of a configuration that can be used with the embodiments described herein. In some examples, one or more of the scanning elements may include one or more of: an oscillating plane mirror, a galvanometer mirror, a MEMS mirror, a rotating mirror, prism or polygon scanner, and/or a resonant mirror, for example.

The mask 206 is provided which is arranged to block a portion P₂₀₁ of the beam 201 reflected from the polygon scanning mirror 202 which has been reflected back from at least one of the cornea C of the eye E and/or an optical element of the optical system 220 from reaching the detector 205, as explained above. The optical elements of the example of the optical system 222 which may reflect back the portion P₂₀₁ of the beam 201 towards the detector 205 include, but are not limited to, the plurality of further scan relay elements shown in Figure 4.

In some examples, the detector 205 may be a separate component or device that is operatively coupled with the light source 200 such that the detector 205 and the light source 200 may be positioned at the same location or adjacent to one another. In some examples, the light source 200 may be implemented with the detector 205 as a single device, or devices within a common housing, such that in addition to emitting the scanning beam 201, the device may also be capable of detecting or receiving light, such as the light reflected back from the first scanning element 202 or from the optical element 204. In some further examples, the ophthalmic imaging instrument 120 may include a focussing lens placed before the detector 205 which is arranged to focus all, or substantially all, of the returning light L_{R} reflected from the rotating polygon mirror 202 onto the detector 205.

In an exemplary SLO imaging modality, the scanning beam 201 may be a laser of suitable wavelength as used in SLO applications. If other imaging modalities are included, the scanning beam 201 may be a collimated light which includes the laser for SLO applications and a superluminescent diode (SLD) for other applications, for example. It should be appreciated that any suitable source of collimated light may be used, such as a single frequency laser diode, vertical-cavity surface-emitting laser, wavelength swept laser source, pulsed laser source, or other source that has enough intensity and to be well collimated and produce adequate retinal illumination. In such applications, the SLD may be used due to the short coherence lengths required to discriminate the retinal layers from the resultant interferometric data. The SLD may be free space or fibre coupled into standard or polarization maintaining fibre to the scan system. A swept source laser may also be used in other applications, whereby the wavelength of the source is tuned over a given range.

In some embodiments, each of the first scanning element 202 or the second scanning element 206 may be a single element or an arrangement of two or more elements as suitable to provide a scan at a respective focal point F1 or F2, as shown, at which the scanning element is disposed. The focal points F1 and F2 are the foci of the optical element 204, and the focal points F2 and F3 are the foci of the optical element 208. The first scanning element 202 is positioned at focal point F1, the second scanning element 206 is positioned at focal point F2, and the eye E (e.g. the pupil of the eye E) is positioned at focal point F3 (also referred to as a virtual scanning point). The resulting scan may be a 2D scan or scan pattern of the scanning beam 201 as the light sweeps through the virtual scanning point (e.g. through F3) onto the fundus F of the eye E, thereby passing through the cornea C of the eye E.

In some embodiments, the first scanning element 202 provides either a vertical, horizontal, or patterned scan which is incident on the optical element 204, to a point on the second scanning element 206 via the optical element 204. The scans may be 1D or 2D light scans, for example. The axes of the first scanning element 202 and the second scanning element 206 may be arranged to create a 2D light scan, such as in the form of a raster scan pattern of the scanning beam 201. The alignment of the first and second scanning elements 202, 206 may be orthogonal, substantially orthogonal, or arranged to generate an arbitrary scan geometry about the optical elements 204 and 208.

In some embodiments, the second scanning element 206 provides a plurality of scans, for example 1D or 2D light scans, which may comprise horizontal scans, vertical scans, or arbitrary patterns of the scanning beam 201. The scans provided by the first scanning element 202 and the scans provided by the second scanning element 206 are different from each other, for example with respect to the orientation of the scans. In some examples, one of the scanning elements may provide vertical scans of the retina, and the other scanning element may provide horizontal scans of the retina. The scanning beam 201 is directed towards the eye E via the scanning elements 202 and 206, and the optical elements 204 and 208, such that, for example, a wide field, or an ultra-wide field, scan angle is achieved at the pupil plane of the eye E.

A "wide field" scanning refers to a scan angle in excess of 50 degrees in one or two dimensions. An "ultra-wide field" scanning refers to a scan covering substantially the entire retina of the eye E. In some examples, the plurality of line scans may be generated by scanning the retina along a first direction using the first scanning element 202, and positions of the plurality of line scans are varied along a second direction using the second scanning element 206, where the second direction is orthogonal to the first direction. As illustrated in Figure 4, the path of the scanning beam 201 is shown in a 1D scan produced by one oscillation or rotation (shown by the curved arrow) of the first scanning element 202. Path "A" is an example of the scanning beam 201 reflected from the polygon scanning mirror at one orientation of a reflecting facet during rotation, whereas paths "B" and "C" are examples of the scanning beam 201 reflected at other orientations of the facet during rotation.

The components of the ophthalmic imaging instrument 120 may be arranged such that the rotational axis of the first scanning element 202 is substantially parallel to a line joining the two foci of the optical element 208 (i.e., F2 and F3) such that the scanning beam 201 is scanned across the secondary axis of the optical element 204. Furthermore, the first scanning element 202 may produce a 1D or 2D scan which is incident on the optical element 204. The optical element 204 may also therefore produce a 1D or 2D scan. The components of the ophthalmic imaging instrument 102 may be arranged such that the line joining the two foci of the optical element 208 (i.e., F2 and F3) lies substantially on a plane defined by the scan (e.g., 1D vertical scan) produced by the optical element 204.

The first and second scanning elements 202 and 206 may thus together create a light scan, for example a 2D scan, in the form of a raster scan pattern from a single point in space at or near the focal point F3 at or in the eye E of the patient. The first and second scanning elements may have operating parameters which include the amplitude of the oscillation and the rotational offset of the oscillation. The operating parameters also include the velocity of oscillation. Both of these operating parameters may be selected to control the direction and pattern of the light scan from the apparent point source. In some examples, the first and second scanning elements may be housed in a rotation mount (not shown) that can adjust the centring (or eccentricity) of the scanning beam 201 on the fundus F of the eye E, which provides the ability to "move" the imaging field across the fundus F.

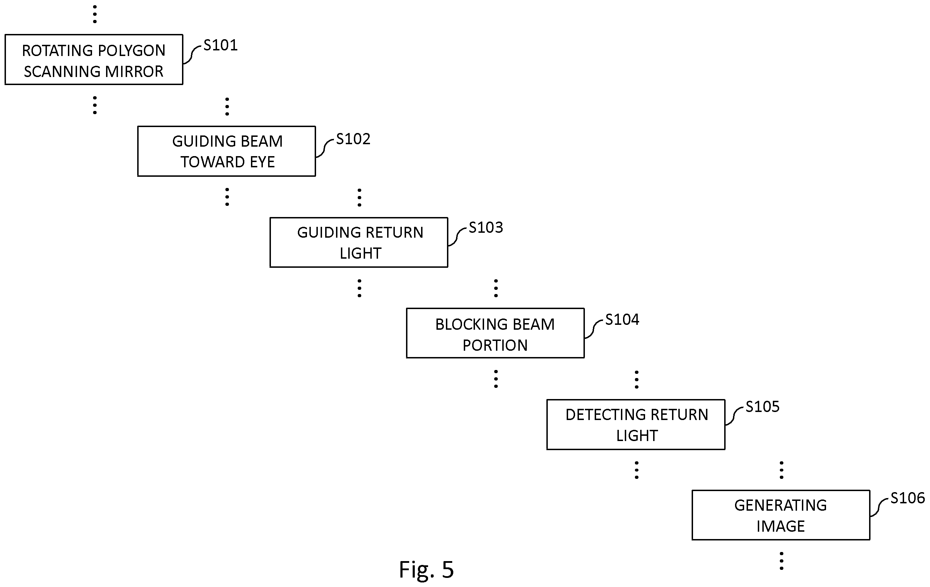

Figure 5 shows a flowchart of a general method embodiment of the present invention. Specifically, the method embodiments relate to operating an ophthalmic imaging instrument that comprises a light source, a polygon scanning mirror, an optical system, a mask, a detector and an access to computing resources. For example, the methods may be suitable for operating the ophthalmic imaging instrument 102 or 120 as disclosed and described in conjunctions with embodiments of the present disclosure. The method comprises a step S101 of rotating, by means of the driver, said polygon scanning mirror such that each facet reflects a beam of light, a step S102 of guiding, by means of the optical system, the beam reflected from the polygon scanning mirror towards a fundus of an eye of the subject through a cornea of the eye, a step S103 of guiding, by means of the optical system, light returning from the fundus towards the polygon scanning mirror, a step S104 of blocking, by means of the mask, a portion of the beam reflected from the polygon scanning mirror which has been reflected back from at least one of the cornea of the eye and/or an optical element of the optical system (along an optical path which would be detected by the detector) from reaching the detector (so as to at least partially suppress, or remove, an image artifact in the image of the fundus which would result from detecting the portion of the beam when generating the detection signal), a step S105 of generating, by means of the detector, the detection signal by detecting the returning light (reflected from the polygon scanning mirror) and a step S106 of generating, by means of the computing resources (e.g. via the access), the image of the fundus of the eye using the detection signal. It is noted that the steps S101 to S106 can be performed concurrently and/or at least in part sequentially as is most suitable for the chosen implementation.

The forgoing has presented embodiments and details thereof as part of the present invention which can provide one or more advantages by improving power control mechanisms in ophthalmic imaging instruments that allow for the flexibility of providing a plurality of imaging modalities but maintain safety while not requiring an increase in system and/or implementation. While various embodiments of the present disclosure have been described above, it should be understood that they have been presented by way of example, not limitation. It will be apparent to persons skilled in the relevant art(s) that various changes in form and detail can be made therein. Thus, the above-described exemplary embodiments are not limiting.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

## Claims

1. An ophthalmic imaging instrument (102, 120) comprising:
a light source (200) arranged to emit a beam of light (201);
a polygon scanning mirror (202) comprising a plurality of reflecting facets (2021, 2022, ...);
a driver (203) arranged to rotate during operation said polygon scanning mirror (202), such that each facet reflects the beam of light (201);an optical system (220) arranged to guide the beam (201) reflected from the polygon scanning mirror (202) towards a fundus (F) of an eye (E) of a subject through a cornea (C) of the eye (E) and to guide light (L_{R}) returning from the fundus (F) towards the polygon scanning mirror (202);
a mask (206) arranged to block a portion (P₂₀₁) of the beam (201) reflected from the polygon scanning mirror (202) which has been reflected back from at least one of the cornea (C) of the eye (E) and an optical element (204, 206, 208) of the optical system (220) from reaching a detector (205);
the detector (205) arranged to detect the returning light (L_{R}) reflected from the polygon scanning mirror (202) to generate a detection signal (S_{d}); and
an access (207) to computing resources (106) which are arranged to generate an image (210) of the fundus (F) of the eye (E) using the detection signal (S_{d}).

2. The ophthalmic imaging instrument (102, 120) of Claim 1, wherein the portion (P₂₀₁) of the beam of light (201) is blocked by the mask (206) from being incident on a portion of at least one facet of the polygon scanning mirror (202) that is not presently reflecting the beam of light (201) during rotation of the polygon scanning mirror (202) whereby the detected returning light (L_{R}) forms the image (210).

3. The ophthalmic imaging instrument (102, 120) of Claim 2, wherein the portion (P₂₀₁) of the beam of light (201) is blocked by the mask (206) from being incident on a portion of a first facet (2022, 2023) of the polygon scanning mirror (202) which is the next to reflect, or the last that reflected, the beam of light (201) during rotation of the polygon scanning mirror (202) whereby the detected returning light (L_{R}) forms the image (210).

4. The ophthalmic imaging instrument (102, 120) of Claim 3, wherein the portion (P₂₀₁) of the beam of light (201) is blocked by the mask (206) from being incident on the first facet (2022, 2023) for all optical paths of the portion (P₂₀₁) of the beam of light (201) which would be incident on the first facet (2022, 2023) during rotation of the polygon scanning mirror (202) whereby the returning light (L_{R}) forms the image (201).

5. The ophthalmic imaging instrument (102, 120) of Claim 3 or 4, wherein the first facet (2022) of the polygon scanning mirror (202) shares an edge (E) with an active facet (2021) presently reflecting the beam of light (201) and wherein an obtuse angle (θ) is formed between the beam of light (201) and a line from the edge (E) to the point of incidence of the beam of light (201) on the active facet (2021).

6. The ophthalmic imaging instrument (102, 120) of any preceding claim, wherein the polygon scanning mirror (202) is enclosed by a housing (208) with an opening (209), where the opening (209) extends across a plane (211).

7. The ophthalmic imaging instrument (102, 120) of Claim 6, wherein the opening (209) comprises a phase mask (210).

8. The ophthalmic imaging apparatus (102, 120) of Claim 6 or 7, wherein the mask (206) is fixed within the housing (208).

9. The ophthalmic imaging instrument (102, 120) of any of Claims 6 to 8 when dependent on Claim 5, wherein the portion (P₂₀₁) of the beam of light (201) is blocked by the mask (206) from being incident on the first facet (2022) by the mask (206) blocking the opening (209) of the housing (208) up to the edge (E) when the active facet (2021) is parallel to the plane (211).

10. The ophthalmic imaging apparatus (102, 120) of any preceding claim, wherein each of the plurality of facets (2021, 2022, ...) extend a first distance in a direction orthogonal to the plane of rotation of the polygon scanning mirror (202) and the mask (206) extends a second distance equal to or greater than the first distance in the direction orthogonal to the plane of rotation of the polygon scanning mirror (202).

11. The ophthalmic imaging instrument (102, 120) of any preceding claim, wherein a surface (212) of the mask (206) blocks the portion (P₂₀₁) of the beam (201) and wherein each facet of the polygon scanning mirror (202) is parallel to the surface (212) of the mask (206) at a given angular rotation of the polygon scanning mirror (202).

12. The ophthalmic imaging instrument (102, 120) of any preceding claim, wherein the image (210) of the fundus (F) of the eye (E) is generated using the detection signal (S_{d}) generated from the returning light (L_{R}) reflected by all facets (2021, 2022, ...) during rotation of the polygon scanning mirror (202).

13. The ophthalmic imaging instrument (102, 120) of any preceding claim, wherein the polygon scanning mirror (202) is a regular convex polygon with 16 facets.

14. The ophthalmic imaging instrument (102, 120) of any preceding claim, wherein the ophthalmic imaging instrument (102, 120) is a scanning laser ophthalmoscope.

15. A method for operating an ophthalmic imaging instrument (102, 120) according to any preceding claim, the method comprising:
- rotating (S101), by means of the driver (203), said polygon scanning mirror (202) such that each facet (2021, 2022, ...) reflects the beam of light (201);
- guiding (S102), by means of the optical system (220), the beam (201) reflected from the polygon scanning mirror (202) towards the fundus (F) of the eye (E) of the subject;
- guiding (S103), by means of the optical system (220), light (L_{R}) returning from the eye (E) towards the polygon scanning mirror (202);
- blocking (S104), by means of the mask (206), the portion (P₂₀₁) of the beam (201) reflected from the polygon scanning mirror (202) from reaching the detector (205);
- generating (S105), by means of the detector (205), the detection signal (S_{d}) by detecting the returning light (L_{R}); and
- generating (S106), by means of the computing resources (106), the image (210) of the fundus (F) of the eye (E) using the detection signal (S_{d}).
